# FASCICULE DE BREVET EUROPEEN

(11) **EP 0 594 770 B1**
(45) Date de publication et mention de la délivrance du brevet: **15.01.1997**
(21) Numéro de dépôt: 92916114.9
(22) Date de dépôt: 17.07.1992
(51) Int. Cl.: A61K 31/54, A61K 9/08, A61K 47/32

(54) **COMPOSITIONS OPHTALMIQUES A BASE D'OXICAM OU DE DERIVES D'OXICAM**
OXICAM UND OXICAMDERIVATE ENTHALTENDE OPHTHALMISCHE ZUSAMMENSETZUNGEN
OXICAM OR OXICAM DERIVATIVES-CONTAINING OPHTHALMIC COMPOSITIONS

(30) Priorité: 18.07.1991 FR 9109096
(43) Date de publication de la demande: 04.05.1994
(73) Titulaire: LABORATOIRE EUROPHTA S.A., 98000 Monaco (MC)
(72) Inventeur: SCHWADROHN, Gérard, F-06000 Nice (FR)
(74) Mandataire: Burtin, Jean-François
(86) Numéro de dépôt international: FR9200696
(87) Numéro de publication internationale: WO9301814

(56) Documents cités:
- EP-A- 0 101 178
- GB-A- 2 007 091

## Description

La présente invention concerne de nouvelles compositins pharmaceutiques destinées à l'usage ophtalmique ainsi qu'à leurs procédés de préparation.

Elle a plus particulièrement pour objet de nouvelles compositions destinées à l'instillation oculaire et présentant des propriétés d'adhérence et/ou de remanence très améliorées.

On sait, en effet, que lors de l'instillation de préparations ophtalmiques une quantité, non négligeable, de cette préparation s'écoule spontanément de l'oeil, soit qu'elles soient mal versées ou que le réflexe palpébral entraine l'évacuation de la préparation. Une autre partie de l'instillation dans l'oeil coule dans le canal lacrymal et par là, s'écoule dans les fosses nasales. C'est pourquoi, on estime que l'administration d'un collyre ou de toute autre préparation ophtalmique conduit à une déperdition très importante de principe actif. Ce phénomène est encore plus marqué quand il faut administrer un tel médicament à de jeunes enfants plus ou moins rétifs ou à des personnes âgées dont les mouvements sont maladroits.

Pour remédier à une telle situation, on peut avoir recours à des solutions plus concentrées en pensant que le petit volume qui demeurera au contact du globe oculaire ou du sac lacrymal atteindra l'organe cible et aura une meilleure efficacité. Cependant, ces solutions ophtalmiques ont des inconvénients encore plus grands que les solutions diluées. Les solutions hypertoniques sont douloureuses. Les solutions trop concentrées laissent des dépôts sur le globe oculaire et gênent la vision. Les solutions concentrées ont, en outre, l'inconvénient d'entrainer plus fréquemment des effets secondaires indésirables liés à un effet systémique.

On a déjà eu également recours à des solutés hypervisqueux que l'on applique sur la paupière ou sur le globe oculaire. De telles préparations sont décrites dans le brevet français FR-A-2.407.714 (TOKO YAKUHIN KOGYO) ou dans le brevet européen EP-A-166.061 et sont des gels dont la viscosité s'échelonne de 1.000 à 100.000 centipoises à 20°C. En fait, ce sont des pommades ophtalmiques dont l'excipient est aqueux. Il n'est pas certain que de telles préparations puissent s'étaler aisément sur l'oeil, surtout en présence d'électrolytes dont le but est de retarder la rupture du gel. De ce fait, ils ne peuvent trouver d'emploi que pour le traitement des infections des paupières ou du bord externe de l'oeil.

L'objet de la présente invention est de remédier aux inconvénients décrits ci-dessus, en réalisant une préparation instillable, stérile, c'est-à-dire une préparation que l'on peut administrer sous forme de gouttes dans l'oeil, et qui néanmoins, possède une viscosité déjà importante dûe à un polymère dont la nature assure une meilleure adhésivité et par suite une remanence du principe actif plus prolongée.

La présente invention se rapporte donc à de nouvelles compositions ophtalmiques de viscosité moyenne caractérisée en ce qu'elles renferment comme principe actif un agent anti-inflammatoire choisi dans le groupe des oxicams associé à un polymère d'acide acrylique gélifiable et à un agent basique. Le but à réaliser en particulier avec les agents anti-inflammatoires est d'assurer une meilleure biodisponibilité grâce au fait que le soluté aqueux résultant possède à la fin, une fluidité suffisante tout en manifestant un pH compris entre 6,5 et 8 où se produit la gélification maximale.

Pour les agents anti-inflammatoires du type oxicam, le rôle du Carbopol est d'assurer la remanence du principe actif et l'adhésion à l'oeil en assurant ainsi un meilleur contact avec l'organe-cible.

On a constaté, en effet, qu'en utilisant un polymère d'acide acrylique, on obtient un retour à un pH compatible avec le milieu utilisable en ophtalmologie tout en conservant le principe actif sous une forme dissoute.

L'invention est également caractérisée par le fait que le polymère gélifiant est un polymère d'acide acrylique ou Carbomère, notamment ceux commercialisés sous les dénominations Carbopol 934, 934 P, 940 et 941 (Goodrich).

Le polymère d'acide acrylique est ajouté au milieu en quantités s'échelonnant entre 0,05 et 5% en poids et de préférence entre 0,1 et 1% de façon à ce qu'après neutralisation ou quasi-neutralisation par un agent basique, la viscosité du milieu reste dans les limites moyennes fixées à l'avance. On sait, en effet, que les polymères d'acide acrylique donnent des solutions fluides à pH acide qui deviennent visqueuses à partir de pH 5, jusqu'à former des gels solides, puis donnent à nouveau des solutions fluides lorsque le pH est supérieur à 10. L'ajustement de la viscosité des solutions de polymère d'acide acrylique est donc un problème difficile. Il se fait soit par variation des concentrations en polymère, soit par ajustement du pH dans des zones de forte viscosité ou au contraire, dans des zones de faible viscosité mais compatible avec les conditions physiologiques et la stabilité du principe actif.

Dans les préparations selon la présente invention, le problème de la viscosité a été résolu en utilisant des solutions de polymères d'acide acrylique très diluées. De cette façon, il est possible d'ajuster le pH de la préparation au voisinage de la neutralité qui est la zone la mieux tolérée par le milieu oculaire et/ou à une zone de pH, appropriée avec la stabilité du principe actif.

L'agent basique qui sert à ajuster le pH et par voie de conséquence à créer la viscosité de la solution initiale est une base minérale comme un carbonate de métal alcalin ou un bicarbonate de métal alcalin ou bien encore, un hydroxyde de métal alcalin. L'agent basique peut être également une base organique comme une dialcoylamine comme la diethylamine, une trialcoylamine comme la triethylamine ou une hydroxy alcoylamine comme l'éthanolamine, la triethanolamine, la N-méthylglucamine ou le trométhanol.

D'une manière préférée, l'agent basique est l'hydroxyde de sodium ou l'hydroxyde de lithium ajouté à doses calculées pour que le pH final de la solution se trouve dans la zone désirée et compatible avec l'utilisation ophtalmique.

Les préparations ophtalmiques selon l'invention contiennent en outre un agent conservateur comme par exemple un acide-phénol ou un de ses esters comme les parabens ou les gallates.

L'agent conservateur peut être également un sel d'ammonium quaternaire comme le bromure de cetrimide, le chlorure de benzalkonium ou bien le bromure de benzododecinium.

Il peut être avantageux d'ajouter à la préparation, un agent complexant pour assurer une meilleure stabilité de la molécule lorsque le principe actif est sensible à la présence de traces métalliques susceptibles de favoriser sa dégradation. On utilisera, à cette fin, l'acide nitrilo triacétique, l'acide éthylène diamino tétracétique ou l'acide éthylène diamino NN'-diacétique NN'-dipropionique.

Le rôle de l'agent chelatant est également de favoriser la complexation des ions divalents (calcium, magnesium) qui ont une influence défavorable sur l'évolution de la forme galénique pendant la durée du stockage.

Les préparations ophtalmiques selon l'invention contiennent également un agent isotonisant, de préférence une molécule organique apte à ajuster la concentration molaire au voisinage de l'isotonie.

Les agents isotonisants les plus appropriés sont les polyols comme par exemple le manitol, le sorbitol, le xylitol, le dulcitol, le lactitol ou le charmitol qui permettent grâce à leur poids moléculaire élevé, d'ajuster très précisement la quantité d'agent isotonisant requise.

Le principe actif est un agent anti-inflammatoire du type Oxicam qui est susceptible de réduire la synthèse des prostaglandines par inhibition des prostaglandines-synthétases et/ou des cyclo-oxygénases. On sait, en effet, que la perfusion veineuse de prostaglandines (PG A₂ ou PG E₁) augmente sensiblement la pression intra-oculaire. On utilise donc des collyres renfermant des anti-inflammatoires pour inhiber la montée de la pression intra oculaire. Ils sont également actifs pour inhiber la synthèse des leucotriènes et des lipoxines.

Les agent anti-inflammatoires convenant pour cet objectif sont notamment le meloxicam, l'isoxicam, le droxicam, le piroxicam, l'oxicam, le ténoxicam ou le sudoxicam. Ces composés peuvent être définis commodément par la formule générale 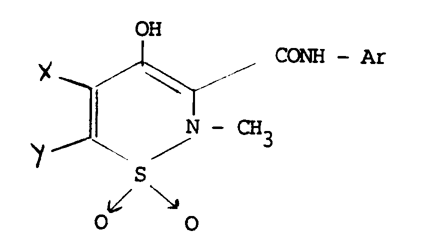 dans laquelle X et Y forment ensemble un cycle aromatique mono cyclique éventuellement substitué
et Ar est un radical phényle, éventuellement substitué, hétéro cyclique comme pyridyle, 5-méthyl 3-isoxazolyl.

Ces composés se comportent comme des acides faibles, par suite de l'ionisation de la fonction phénol et peuvent, de ce fait, passer en solution en milieu alcalin.

Ils sont utilisés sous forme aci ou plus commodement sous forme de sels avec une base minérale (sel de sodium) ou organique (sel de trométhanol, sel de lysine, sel de N-méthyl glucamine ou sel de mono éthanolamine). La concentration en agent anti-inflammatoire s'échelonne de 0,01 à 0,5% et de préférence de 0,05 à 0,2% selon leur niveau d'activité.

Les préparations selon l'invention peuvent également renfermer un agent anti-bactérien et en particulier un agent anti-bactérien du type antibiotique ou du type des quinolones. On citera donc comme anti-bactériens, plus particulièrement préférés, le chroramphénicol, le thiamphénicol, la polymixine, la thyrocidine, la gramicidine, le colistine méthane sulfonate de sodium, la tétracycline, la pénicilline, la néomycine, la gentamicine, la dihydrostreptomycine, l'amikacine, la kanamycine, la dibekacine, la micronomycine, la phosphomycine, la rifamycine ou le sulbactame; l'agent anti-bactérien peut être aussi bien un agent synthétique du type quinolone comme la norfloxacine, la péfloxacine, l'ofloxacine, l'amifloxacine ou la ciprofloxacine.

L'invention concerne également un procédé de préparation des préparations ophtalmiques instillables selon l'invention qui consiste en ce que l'on dissout ou disperse le polymère d'acide acrylique dans un milieu aqueux auquel on ajoute ensuite l'agent conservateur, l'agent isotonisant et l'agent chelatant préalablement dissouts dans un milieu aqueux, puis on incorpore à cette solution aqueuse la solution ou la suspension aqueuse de principe actif puis une solution d'agent basique jusqu'à l'obtention d'un mélange visqueux homogène.

On peut également incorporer directement le principe actif à une des solutions aqueuses. Chacune des solutions ou dispersions ou le principe actif lui-même sont stérilisés par les moyens physiques appropriés. Le mélange des solutions est effectué ensuite.

Les compositions pharmaceutiques selon l'invention peuvent contenir, en outre, d'autres principes actifs comme par exemple un agent anti-bactérien.

Les compositions ophtalmiques instillables selon l'invention trouvent un emploi dans le traitement des affections oculaires notamment dans le syndrome de l'oeil rouge ou les infections oculaires d'origine microbiennes ou virales, les phénomènes d'allergie, les inflammations, le traitement des kératites associé ou non à une infection oculaire, notamment pour les soins préopératoires pour la préparation du patient. La posologie va de 1 à 5 gouttes dans chaque oeil, par jour, répartie en autant d'instillations.

La présence de polymère d'acide acrylique dans la formulation conduit à une réduction du nombre d'instillations et à une diminution de la concentration en principe actif.

Les exemples suivants illustrent l'invention sans toutefois la limiter :

### EXEMPLE I

### PREPARATION INSTILLABLE A BASE DE PIROXICAM

| | |
|---|---|
| Polymère d'acide acrylique commercialisé sous la dénomination Carpobol 940 (Goodrich) | 7,50 g |
| Ethylène diaminotetracetate de sodium | 0,50 g |
| Bromure de cetrimide | 0,50 g |
| Sorbitol | 200,00 g |
| Piroxicam | 5,00 g |
| Hydroxyde de sodium | 4,00 g |
| Eau | 4783,00 g |
| pour un total de 5.000 g environ | |

### MODE OPERATOIRE

### a) Préparation de la solution de polymère d'acide acrylique

Dans un ballon de 5 l, on introduit 750 ml d'eau et sous agitation de 500 t/mn environ, on ajoute peu à peu 7,50 g de polymère d'acide acrylique et on poursuit l'agitation jusqu'à obtention d'une solution homogène sans grumeaux.

### b) Solution de tétracémate de sodium

Dans 1.000 ml d'eau, on ajoute successivement et sous agitation le tétracémate de sodium (0,50 g) puis le bromure de cétrimide (0,50 g) et enfin, le sorbitol (200 g).

On transvase dans le ballon de 5 l cette solution, on rince le récipient avec 250 ml d'eau et on homogénéise.

La solution totale est transférée dans une cuve. On rince à nouveau le ballon avec 250 ml d'eau et on stérilise le mélange à la chaleur.

### PREPARATION DE LA SOLUTION DE NEUTRALISATION

Dans 40 ml d'eau, on dissout sous agitation 4 g d'hydroxyde de sodium. On met 33 ml de la solution ainsi réalisée dans la chambre de pression pour filtration ultérieure.

Dans un bécher contenant 1993 ml d'eau, on introduit 7 ml restants de la solution de soude préparée immédiatement auparavant sous agitation. On homogénéise la solution dont on vérifie le pH (pH = 12,2) et que l'on fait passer progressivement dans un bécher contenant 5,00 g de piroxicam exactement pesés. On maintient l'agitation presqu'à l'achèvement de l'addition. Le piroxicam se dissout et la solution ainsi réalisée à un pH de 10,0.

Dans la cuve, en procédant sous agitation, on filtre stérilement les 33 ml de solution de soude pour neutralisation sur filtre stérilisant et sous pression d'azote stérile.

On rince par ailleurs le bécher et le filtre, avec 250 ml d'eau.

Dans les mêmes conditions, on filtre sur filtre stérilisant dans des conditions de stérilité, la solution de piroxicam et on rince le récipient. On la fait passer dans la cuve contenant déjà le polymère d'acide acrylique, on homogénéise pendant une heure puis on prélève stérilement un échantillon pour vérifier le pH (7.4 à 7.8). On laisse reposer 24 heures puis on homogénéise encore 10 mn puis on prélève à nouveau un échantillon dont on vérifie le pH et la viscosité. La solution visqueuse est prête pour le conditionnement en flacons unidoses de 0,2 ml.

En opérant de la même façon, on peut réaliser un soluté ophtalmique à base de sudoxicam à 2% ou un soluté ophtalmique à base de Tenoxicam à 1%.

### EXEMPLE II

### PREPARATION INSTILLABLE A BASE DE PIROXICAM ET DE POLYMYXINE

| | |
|---|---|
| Polymère d'acide acrylique commercialisé sous la dénomination Carpobol 940 (Goodrich) | 7,50 g |
| Ethylène diaminotetracetate de sodium | 0,50 g |
| Chlorure de benzalkonium | 0,125 g |
| Piroxicam | 5,00 g |
| Polymyxine sous forme de sulfate | 750.000.000 U |
| Hydroxyde de sodium | 4,00 g |
| Chlorure de sodium | 40,00 g |
| Eau q.s.p | 5000 g environ |

La polymyxine peut être remplacée par une quantité équivalente de ciprofloxacine (15 g de principe actif).

## Revendications

1. De nouvelles compositions ophtalmiques à viscosité moyenne instillables, caractérisées en ce qu'elles renferment comme principe actif un agent anti-inflammatoire du groupe des oxicams en combinaison avec un polymère d'acide acrylique gélifiant et un agent basique, de façon à ce que le soluté aqueux en résultant possède une fluidité satisfaisante et un pH compris entre 6,5 et 8.

2. Une composition ophtalmique selon la revendication 1° dans laquelle le polymère d'acide acrylique est un carbomère commercialisé sous les dénominations Carbopol 934, 934 P, 940 et 941 (Goodrich).

3. Une composition opthalmique selon la revendication 1° et la revendication 2° dans laquelle la concentration de polymère d'acide acrylique s'échelonne entre 0,05 et 5 g %.

4. Une composition ophtalmique selon l'une des revendications 1 à 3° dans laquelle la concentration de polymère d'acide acrylique est de l'ordre de 0.1%.

5. Une composition ophtalmique selon l'une des revendications 1 à 4° das laquelle l'agent basique est une base minérale ou organique.

6. Une composition ophtalmique selon l'une des revendications 1 à 5° dans laquelle la quantité d'agent basique est déterminée pour un pH final de la composition s'échelonnant entre 7,0 et 8,0.

7. Une composition ophtalmique dans laquelle le pH est compris entre 7,4 et 7,8.

8. Une composition ophtalmique selon l'une des revendications 1 à 6° dans laquelle l'agent anti-inflammatoire est choisi parmi le droxicam, piroxicam, le sudoxicam, l'oxicam, le tenoxicam, l'isoxicam et le Meloxicam.

9. Une composition ophtalmique selon la revendication 8° dans laquelle la concentration en principe actif s'échelonne de 0,01 à 0,5 %.

10. Une composition pharmaceutique selon l'une des revendications 1 à 7° qui contient, en outre, un agent antibactérien choisi dans le groupe des antibiotiques et dans le groupe des quinolones.

11. Un procédé d'obtention des compositions ophtalmiques selon l'une des revendications 1 à 8° dans lequel on disperse le polymère d'acide acrylique dans un milieu aqueux puis on aoute le ou les autres excipients en solution dans un milieu aqueux en solution ou en suspension dans un milieu aqueux et enfin l'agent basique contenant l'agent anti-inflammatoire et les autres principes actifs en solution ou en suspension dans ledit milieu aqueux jusqu'à obtention d'un mélange visqueux homogène.

## Claims

1. Novel ophtalmologic compositions with medium viscosity, which can be instilled, characterized in that they contain as active ingredient, an anti-inflammatory drug of the oxicam group, in combination with a gelifying polymer of acrylic acid and a basic agent, in order as the resulting aqueous solute possesses a satisfactory fluidity and a pH value between 5, 6 and 8.

2. An ophtalmologic composition according to claim 1 wherein the acrylic acid polymer is a carbomer sold under the trade denominations Carbopol 934, Carbopol 934 P, Carbopol 940 and Carbopol 941 (Goodrich).

3. An ophtalmologic composition according to claims 1 and 2 wherein the concentration in acrylic acid polymer ranges from 0,05 and 50 %.

4. An ophtalmologic composition according to one of claims 1 to 3 wherein the concentration in acrylic acid polymer is of the order of 0,1 %.

5. An ophtalmologic composition according to one of the claims 1 to 4 wherein the basic agent is a mineral or organic base.

6. An ophtalmologic composition according to one of the claims 1 to 5 wherein the amount of basic agent is determined for a final pH value of the composition ranging from 7,0 to 8,0.

7. An ophtalmologic composition wherein the pH value ranges between 7,4 and 7,8.

8. An ophtalmologic composition according to one of the claims 1 to 6 wherein the anti-inflamatory agent is selected from the group consisting of droxicam, piroxicam, sudoxicam, oxicam, tenoxicam, isoxicam and meloxicam.

9. An ophtalmologic composition according to claim 8 wherein the concentration in active ingredient ranges from 0,01 an 0,5 %.

10. A pharmaceutical composition according to one of the claims 1 to 7, which further contains an antibacterial agent selected from the group of antibiotics and the group of quinolones.

11. A process for producing the ophtalmologic compositions accordind to one of claims 1 to 8 wherein the acrylic acid polymer is dispersed in an aqueous medium, then the or other diluent(s) are added in solution in an aqueous medium or suspended in an aqueous medium and finally the basic agent containing the anti-inflammatory agent and the other active ingredients in solution or in suspension in the said aqueous medium until a homogeneous viscous mixture is obtained.

## Patentansprüche

1. Neue ophtalmologische Zubereitungen mit mittlere Viskosität, dadurch gekennzeichnet dass sie als Wirkstoff eine Entzündungshemmendemittel aus der gruppe von Oxicame enthalten, zusammen mit einem gelbildende Acrylsaüre Polymer und einem basichen Mittel, so dass die daraus folgende wassrige Lösung eine ausreichende Flussbarkeit ausweist und eine pH Wert zwischen 6,5 und 8 hat.

2. Eine ophtalmologische Zubereitung nach Anspruch 1, worin der Acrylsaüre Polymer ein Carbomer ist, das in der Handel under den Zeichnungen Carbopol 934, 934 P, 940 und 941 (Goodrich) gestellt ist.

3. Eine ophtalmologische Zubereitung nach Ansprüche 1 und 2, worin das Gehalt an Acrylsaüre Polymer zwischen 0,05 und 5 g % erstreckt.

4. Eine ophtalmologische Zubereitung nach einer der Ansprüche 1 bis 3, worin das Gehalt an Acrylsaüre Polymer um 0,1 % ist.

5. Eine ophtalmologische Zubereitung nach einer der Ansprüche 1 bis 4, worin das basiches Mittel eine anorganische oder organische Base ist.

6. Eine ophtalmologische Zubereitung nach einer der Ansprüche 1 bis 5, worin der Anteil des basichen Mittels, für eine endliche pH Wert der Zubereitung bestimmt ist, der zwischen 7,0 und 8,0 liegt.

7. Eine ophtalmologische Zubereitung worin der pH-Wert zwischen 7,4 und 7,8 umfasst wird.

8. Eine ophtalmologische Zubereitung nach einer der Ansprüche 1 bis 6, worin das Entzüdungshemmendemittel aus Droxicam, Piroxicam, Sudoxicam, Oxicam, Tenoxicam, Isoxicam und Meloxicam ausgewählt ist.

9. Eine ophtalmologische Zubereitung nach Anspruch 8, worin das Gehalt an Wirkstoff von 0,01 bis 0,5 % Merstreckt.

10. Eine ophtalmologische Zubereitung nach einer der Ansprüche 1bis 7, die ausserdem ein Antibakterialmittel aus der Gruppe der Antibiotika und der Gruppe der Chinolonen, ausgewählt ist, enthält.

11. Verfahren zur Herstellung der ophtalmologischen Zubereitungen nach einer der Ansprüche 1bis 8, worin man das Acrylsaüre Polymer in eine wassrige medium dispergiet, denn man den oder die andere Träger in Auflösung in einer schliesslich das das Entzündungshemmendemittel und die weitere Wirkstoff enthalten de basiches mittel in einer Lösung oder in dem genannten wassrigen Mittel, suspendiert bis eine gleichmassige viskose Mischung erhalten wird.
